# EUROPEAN PATENT SPECIFICATION

(11) **EP 1 074 634 B1**
(45) Date of publication and mention of the grant of the patent: **02.10.2002**
(21) Application number: 99401742.4
(22) Date of filing: 09.07.1999
(51) Int. Cl.: C12Q 1/68, G01N 33/68, A61K 38/46, A61K 31/20

(54) **Method for the diagnosis or the prognosis of Alzheimer disease therapeutical composition for preventing or treating Alzheimer disease**
Verfahren zur Diagnose oder zur Prognose von Alzheimer Krankheit: therapeutische Zusammensetzung zur Verhinderung oder zur Behandlung von Alzheimer Krankheit
Procédé pour le diagnostic ou le prognostic de la maladie d'Alzheimer: Composition therapeutique pour la prévention ou le traitement de la maladie d'Alzheimer

(43) Date of publication of application: 07.02.2001
(73) Proprietor: INSTITUT PASTEUR DE LILLE, 59019 Lille Cédex (FR); Centre Hospitalier Universitaire Vaudois de Lausanne, 1011 Lausanne (CH); INSTITUT NATIONAL DE LA SANTE ET DE LA RECHERCHE MEDICALE (INSERM), 75654 Paris Cédex 13 (FR)
(72) Inventor: Mooser, Vincent, 1011 Lausanne (CH); Helbecque, Nicole, 59750 Marcq en Baroeul (FR); Amouyel, Philippe, 59700 Marcq en Baroeul (FR)
(74) Representative: Jacobson, Claude

(56) References cited:
- WO-A-95/24504
- WO-A-96/09392
- WO-A-98/18429
- KUO Y-M, ET AL.: "Elevated low-density lipoprotein in Alzheimer's Disease correlates with brain Abeta 1-42 levels" BIOCHEMICAL AND BIOPHYSICAL RESEARCH COMMUNICATIONS, vol. 252, 1998, pages 711-715, XP002126049
- KURIYAMA M ET AL: "Low levels of aoplipoprotein A1 and A11 in senile dementia" JAPANESE JOURNAL OF PSYCHIATRY AND NEUROLOGY, vol. 48, no. 3, - September 1994 (1994-09) pages 589-93, XP000863586

## Description

The present invention relates to a method for the diagnosis or the prognosis of Alzheimer's disease (AD).

The present invention further provides a therapy for preventing or treating AD.

Alzheimer's disease is a brain pathology characterized by an early dementia with a loss of cortical neurons associated with plaques of β-amyloid, neurofibrillary tangles and, in most cases, an amyloid angiopathy. It is strongly suspected that there is a genetic influence in the aetiology of Alzheimer's disease (WO 94/01772).

This genetic component has been brought to the fore over many years by indirect observations which suggest that the disease is inherited in autosomal dominant fashion with an age-dependant penetrance in order to explain the family links between individuals suffering from the disease. Recent molecular genetic studies have enabled putative genes for Alzheimer's disease to be isolated by looking for chromosome-specific polymorphic genetic markers (Bird et al, 1989, *Neurobiology of Aging* 10, 432-434).

Three chromosomal localizations have been described as being involved in the early onset familial forms (age at onset under 60 years) : chromosome 21, chromosome 14 and chromosome 19. Two linkage studies have suggested that the chromosomal region 19q13.2 was associated with late onset familial forms of Alzheimer's disease (Pericak-Vance et al, *Am. J. Hum. Genet.* (1991), 48, 1034-1050 ; Schellenberg et al, *Ann. Neurol*., (1992), 31, 223-227). Within this chromosomal region, the group of genes for apolipoproteins (APO) E-CI-CI'-CII is a candidate zone. Among the products of these genes, apolipoprotein E (APOE) is involved especially in the nervous system : APOE is present in the senile plaques and possesses a binding affinity for the peptide β-A4. Strittmatter et al (*Proc. Natl. Acad. Sci.* (1993) 90, 177-181) have described an increased frequency of the ε4 allele of the APOE gene in the late onset familial forms of Alzheimer's disease. This observation has been confirmed for the familial forms (Corder et al, *Science* (1993), 261, 921-923) and the sporadic forms of Alzheimer's disease (Corder et al, *Science* (1993), 261, 921-923 ; Saunders et al, *Neurobiology* (1993), 13, 1467-1472).

Moreover, Schellenberg et al (*Ann. Neurol*. 1992, 31:223-227) have reported a genetic association between the F allele of the apolipoprotein Cll gene (Taql restriction fragment length polymorphism (RFLP) allele) and the familial form of Alzheimer's disease.

Furthermore, the instant inventors have already reported in WO 95/24504 that the risk for a subject to develop AD is related to the presence of APO Cll allele or/and the short D19S178 allele together with the APOE ε4.

The authors of the instant invention have further demonstrated that the risk of developing an Alzheimer's disease is increased when a T →G mutation is present in the promoter of the APOE gene leading to a modified expression of the APOE protein.

Furthermore, APO ε4 allele has been associated with coronary atherosclerosis in AD patients (O. Kosunen et al, 1995 ; Stoke, vol. 26, n° 5).

The work of the inventors of the present invention has now led to the discovery that Apo(a) is a risk factor for Alzheimer's disease.

Human Lp(a) is a lipoprotein particle formed by the assembly of a low-density lipoprotein (LDL) particle and a carbohydrate rich highly hydrophobic protein named apolipoprotein (a) [apo(a)]. In Lp(a), one molecule of Apo(a) is covalently linked to the apoB-100 component by a disulfide bridge ; the presence of Apo(a) distinguishes Lp(a) from all other lipoprotein classes.

Besides high carbohydrate content, which constitutes ∼30 % of the protein mass, Apo(a) also exhibits considerable heterogeneity in size and structure. Apo(a) is formed by three distinct structural domains, each exhibiting a high degree of homology with plasminogen. Plasminogen is formed by a protease domain and by five domains called kringles, designated kringle 1 through 5. Each kringle contains six conserved cysteine residues ; these form three disulfide bonds that provide the characteristic triple loop structure of the kringles. Apo(a) contains an inactive protease domain and one copy of kringle 5 domain- both of which exhibit ∼85 % homology with the corresponding domains of plasminogen- and multiple copies of the plasminogenlike kringle 4 (K4) domain. The multiple copies of Apo(a) K4 are similar but not identical to each other and can be divided into 10 distinct kringles types (K4 types through 10) ; their homology with plasminogen K4 ranges between 78 % and 88 %. One copy each of K4 type 1 and types 3 through 10 is present per Apo(a) particle ; K4 type 2, however, is present in a variable number of repeats (from 3 to > 40) which are therefore responsible for the size heterogeneity of Apo(a) and consequently of Lp(a).

The inventors of the instant invention have now examined whether elevated plasma levels of Lp(a) which binds to the same receptors as APOE was associated with AD and found that elevated plasma Lp(a) levels may constitute an additional risk factor for the development of AD in APOE ε4 carriers.

The results of the survey performed by the inventors provide the rational for improving the diagnosis of AD, more precisely predicting an increased risk of developing AD as well as for designing innovative preventive and therapeutic strategies to fight AD.

In one aspect, the present invention provides a method of predicting an increased risk of a patient having Alzheimer's disease or for a subject of developing AD comprising :
a) assaying a DNA-containing biological sample of a patent or a subject for the allele of the APOE gene ;
b) assaying the plasmatic level of protein Lp(a) or glycoprotein Apo(a),
wherein the presence of an ε4 allele of the APOE gene and an increased level of Lp(a)/Apo(a) indicates an increased risk for the patient having Alzheimer's disease or developing AD.

By "increased level of Lp(a)/Apo(a)" in the sense of the instant invention, it is meant that the level of Apo(a) is increased by at least 8.6 mg/dl with respect to a population of same age of healthy subjects.

Any method of measuring the plasmatic level of a protein is suitable.

Preferred embodiments include radioimmunoassays (RIA), enzyme linked immunoassays (ELISA), immunoradiometric assays (IRMA), immunoenzymatic assays (IEMA) fluoroimmunoassays (FIA), chemiluminescent immunoassays, immunoagglutination assays (IA), etc.

The immunoassay may be of different types including sandwich assays using monoclonal and/or polyclonal antibodies.

The immunoassay may be performed in one step, two steps or three steps, as it is known in the art.

The determinations may be performed in liquid phase or on a solid support.

The antibody recognizing Lp(a)/ Apo(a) must be highly specific for Lp(a)/ Apo(a).

In a preferred embodiment, the method of the invention consists of determining the plasmatic level of Lp(a)/Apo(a) by means of a sandwich ELISA method carrying out a first antibody against K4-type 1 and 2 repeats (for capturing Lp(a)) and a second antibody conjugated to horseradish peroxidase directed against K4-type 9 repeats for performing the detection of the immune complex formed thereby.

The determination of the APOE polymorphism is performed according to any method well known in the art.

In one embodiment, this method comprises :
i) amplifying the DNA in a DNA-containing biological sample from a patient or a subject, with a first pair of primers which amplify at least or portion of the APOE gene ;
ii) visualizing the amplification products of step i);
and thereby determining the presence of the APOE ε4 allele.

A "DNA-containing biological sample" refers to a sample of tissue or fluid suspected of containing an analyte polynucleotide from an individual including, but not limited to, e.g. plasma, serum spinal fluid, lymph fluid, the external secretions of the skin, respiratory, intestinal and genitourinary tracts, tears, saliva, blood cells, tumors, organs, tissue and samples of *in vitro* cell culture constituents.

Step i) is performed in a manner known *per se* involving a nucleic acid amplifying method by means of a pair of primers specifically hybridizing to the region flanking the nucleic acid sequence to be amplified.

The method is preferably a DNA amplifying method, namely PCR (Innis et al, 1990 ; PCR protocols : A guide to methods and Applications (Academic Press, San Diego, Californie).

Suitable primers are described in the art and namely include those described by Hixson et al (1990), *J. Lipid Res.* 31, 545-548. Reagents and hardware for conducting PCR are commercially available.

Other suitable methods are well-known and widely practiced in the art (see eg US 4 683 195 and US 4 683 202 and Wu et al, 1989 (Genomics 4 560-569)).

Step ii) is conducted by detecting the hybridization of the amplified products with a polynucleotide probe which forms a stable hybrid with that of the target sequence, under stringent hybridization and wash conditions.

Probes for APOE ε4 are derived from the APOE ε4 allele region and span all or a portion of said region and allow specific hybridization to the APOE ε4 region.

The length of the probe is advantageously of at least 30 nucleotides.

The probes include an isolated polynucleotide attached to a label or a reporter molecule.

According to a further aspect, the instant invention provides a kit for predicting an increased risk of a patient having AD or for a subject of developing AD, wherein the kit comprises :
- a first pair of primers which are capable of amplifying at least a portion of the APOE gene ;
- a reagent for assaying the presence in a DNA sample of the APOE ε4 allele ;
- at least one antibody recognizing Lp(a)/Apo(a).

Advantageously, the reagent is a probe specific for APOE ε4.

The kit may also comprises at least one reagent for amplifying DNA. The reagent is advantageously a polymerase enzyme capable of performing polymerase chain reaction. One example is Taq polymerase.

The kit of the invention may also comprise a reagent for performing polyacrylamide gel electrophoresis.

In a preferred embodiment, the antibody is a monoclonal antibody conjugated to an enzyme, namely horseradish peroxidase.

The present invention further provides a therapy for preventing or treating Alzheimer disease.

Thus, in another aspect, the invention provides the use of a compound capable of decreasing the plasmatic level of Lp(a) for the manufacture of a medicament for preventing or treating Alzheimer disease in a subject bearing at least one APO ε4 allele.

In a first embodiment, the compound is a modifier of the transcription of the Apo(a) gene.

Preferred examples are retinoic acid (*Biochem. Biophys. Res. Commun* 1997, Sept 8, 238(1) :48-52) and danazol (*Biochem. Biophys. Res. Commun* 1996 Oct. 14, 227(2): 570-5).

In a second embodiment, the compound is a modifier of the expression of the Apo(a) gene.

The compound is typically an oligonucleotide ribozyme (*Circulation*, 1998 Nov. 3 (18): 1898-909).

These compounds result in a modified, preferably a decreased synthesis of Apo(a).

In a third embodiment, the compound modifies the folding of the Apo(a) protein.

The compound is typically a compound able to block the formation of disulfide bridges, namely an anti-oxidant.

In a fourth embodiment, the compound modifies the glycosylation of the Apo(a) protein.

One example of that type is catanospermine (*J. Bio*/*Chem*. 1997, Feb. 21, 272 (8) 5078-55).

In a fifth embodiment, the compound modifies the binding of the Apo(a) protein to the cell surface.

The compound is typically an antibiotic, namely of the deoxystreptamine family, such as neomycine (*J. Lipid. Res*. 1996 Oct., 37(10) : 2055-64).

In a sixth embodiment, the compound modifies the binding of Apo(a) to the Apo B100 protein.

The compound may consist of an analog of lysine (for example, epsilon amino caproic acid (*Arterioscler. Thromb. Vasc. Biol*. 1998 Nov., 18(11) : 1738-44), arginine or phenylalanine *(Biochemistry,* 1998 May 26, 37(21) : 7892-8), or of an anti-Apo(a) antibody.

Alternatively, the compound may consist of an antibody recognizing Apo(a) (*J. Biol. Chem*., 1994 Nov. 18, 269(46) :28716-23).

The compounds according to the third, fourth, fifth and sixth embodiment result in a decreased production of Apo(a)/Lp(a).

In a further aspect, the invention provides the use of a compound inhibiting the binding of Apo(a)/Lp(a) to its receptor for the manufacture of a medicament for preventing or treating the Alzheimer disease in a subject bearing at least one APO ε4 allele.

In a first embodiment, the compound is one which blocks the binding site of Apo(a) K4.type 6-7 to the receptor triggered by cholesterol (*J. Biol. Chem.* 1996 Dec.13, 271 (50): 32096-104).

In a second embodiment, the compound is one LPR (low-density lipoprotein receptor related protein).

Typically, the compound consists of an α-2 macroglobulin or a surface proteoglycan, namely heparinase, chondroitinase ABC or sodium chlorate (FASEB J, 1998 Dec., 12(15): 1765-76).

The following non limiting examples and the enclosed figures illustrate the invention.

### LEGENDS OF FIGURES

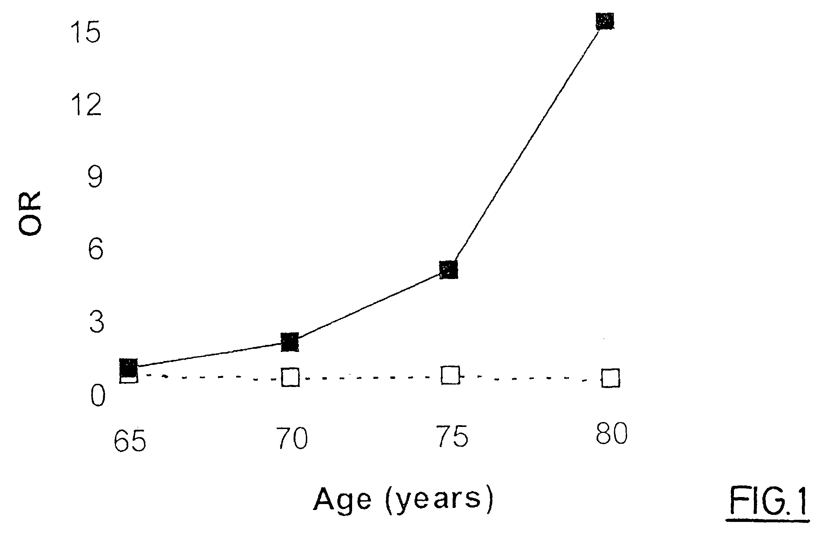

Figure 1 illustrates the relationship between the odds ratio (OR) and the age of onset of patients having AD in presence of plasma Lp(a) levels > 8.6 mg/dl in non-APOE ε4 carriers (broken lane) and in APOE ε4 carriers (solid lane). APOE was genotyped as described in Hixson et al, J. Lipid. Res. 31:545-548) and quantification of plasma Lp(a) levels was performed using an ELISA assay and monoclonal antibodies of well-defined specificity (Marcovina et al, *Clin. Chem*. 1995, 41/2:246-255). The odds ratio were calculated using multivariate logistic regression analysis on log-transformed plasma Lp(a) levels. Confidence intervals are given in the text.

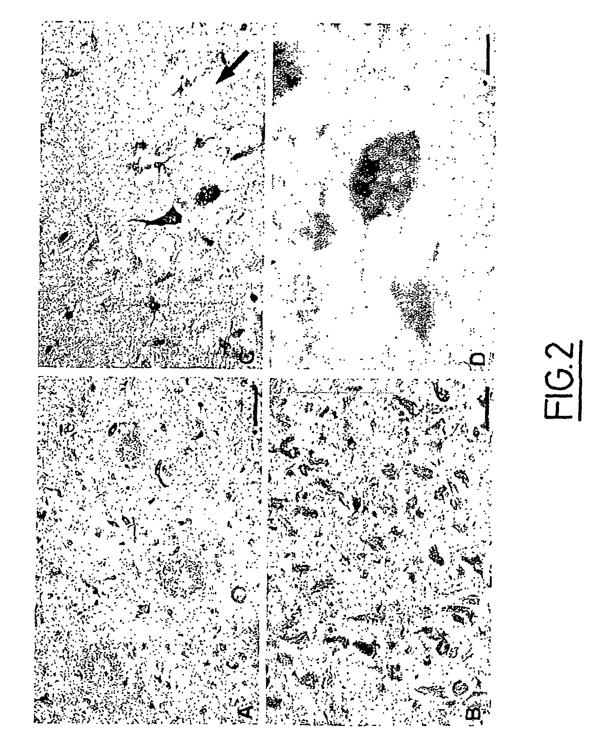

Figure 2 shows the pattern observed in immunodetection of Apo(a) in human brain with AD-type cortical changes. Brains from two subjects with AD-type cortical changes were examined for the presence of Apo(a) using IgG-a5 antibody, as described hereunder in Methods.

### METHODS

### Clinical study

Participants to the study were of Caucasian origin and were recruited in Europe. They were thoroughly investigated clinically. The diagnosis of AD was made to the DSM-III-R criteria (American Psychiatric Association, 1987) and NINCDS-ADRDA (Mc Kahn et al, *Neurol*., 1984, 34:939-944), whereas subjects were categorized as non-demented controls in absence of any DSM-III-R criteria for dementia and the presence of cognitive functions preserved in their integrity. Recruitment of controls took place between February and August, 1993, whereas subjects with AD were recruited between May, 1994 and June, 1995. All female participants were post-menopausal. For each participant, a total of 20 ml of blood was collected on EDTA, stored on ice and subjected to centrifugation within 4 hours. Plasma was isolated and aliquots were stored at -80°C.

### Laboratory methods

Plasma levels of Lp(a) were quantitated on samples which had never been thawed before using an ELISA assay (Marcovina et al, *Clin. Chem.,* 1995, 41/2:246-255). This method utilizes IgG-a6 as capture antibody and IgG-a40 conjugated to horseradish peroxidase as detecting antibody. IgG-6 recognizes the Apo(a) K4-type 1 and 2 repeats. To determine the size of the Apo(a) isoforms, plasma proteins were size-fractionated on a 2 % SDS-agarose gel and the size of the Apo(a) isoforms was determined based on their migration relative to well-characterized standards (Mooser et al, *Am. J. Hum. Gen*. 1997, 61:402-417). In addition, the inventors characterized the (TTTTA)ₙ pentanucleotide repeat polymorphism in the 5' flanking region of the Apo(a) gene (Mooser et al, *Human. Mol. Gen*. 1995, 4:173-181) and performed APOE genotyping (Hixson et al, *J. Lipid.* 1990, 31:545-548).

### Immunohistochemical analysis of Apo(a) in human brain.

A total of 15 autopsy cases were examined. Brains were fixed for 4 weeks in 10 % formalin and ∼ 3x2x1 cm blocks were excised form the frontal and parietal associative areas (Brodmann's areas 8-9 and 39-40, respectively) and from the temporal region. Temporal blocks included the hippocampus and entorhinal cortex. Blocks were embedded in paraffin, and 5 µm sections were used for histological and immunohistochemical analysis. Senile plaques were stained with Thioflavin S technique and examined by immunohistochemistry using a monoclonal antibody (M872) directed against β-amyloid (DAKO, Zug, Switzerland), whereas neurofibrillary tangles were stained using the Gallyas silver technique. Among the 15 autopsy cases examined, ten cases met the quantitative histological criteria for neuropathological diagnosis of AD, as proposed by Khachaturian, (*Arch. Neurol*., 1985, 42:1097-1105), whereas no AD-type cortical changes were detected in the remaining five cases. For immunodetection of Apo(a), sections were deparaffinized, rehydrated and treated with 0.3 % methanolic peroxide for 30 minutes in order to eliminate endogen peroxidase activity. Sections were then treated with diluted normal rabbit serum (1:100) at 37°C for 30 minutes, and incubated overnight at 4°C with IgG-a5 (diluted to 50 µg/ml) or lgG-a40 (diluted to 10 µg/ml). Detection was performed using the ammonium-nickel-sulfate technique, or the ABC kit according to the recommendations of the manufacturer (DAKO), followed by counterstaining with hematoxilin. Control sections were examined after omission of the primary antibody or after incubation with an irrelevant monoclonal antibody.

### Statistical analysis

Statistical analyses were performed with the SAS software release 6.10 (SAS Institute Inc, Cary, NC)). Means and standard deviations (SD) were calculated for quantitative variables. Categorical data were tested using the Pearson's χ2 or Fisher's exact test when necessary. Multivariate logistic regression model was used to estimate adjusted OR and 95 % Cl. Covariates taken into account were age and gender.

### RESULTS

Levels of lipoprotein(a)/[Lp(a)] were quantitated in plasma samples collected from 296 non-demented control individuals and 285 subjects with Alzheimer disease including 200 (70,2 %) subjects who had dementia diagnosed at or after age 65 years (late-onset AD).

Participants were partitioned according to the presence of at least one APOE ε4 allele, and their characteristics are described in the Table hereunder. As expected, the proportion of APOE ε4 carriers among demented subjects (170/285, 60 %) was higher than in the control group [74/296, 25 % ; odds ratio (OR) 4.4, 95 % confidence interval (Cl) 3.1 - 6.4 ; p<0,00001]. The proportion of males was lower than females in the four groups. Demented subjects were older than controls. However, among controls or AD subjects, age was similar for APOE ε4 carriers and non-APOE ε4 carriers.

As expected, in the four groups, the distribution of plasma Lp(a) levels was markedly skewed towards lower values, whereas log-transformed values were more normally distributed.

Among APOE ε4 carriers, plasma Lp(a) levels were higher in subjects with dementia than in controls [9.8 mg/dl vs 6.9 mg/dl (median), p = 0.049, Table] hereunder, with a smaller proportion of subjects with plasma Lp(a) levels < 10 mg/dl (51 % vs 66 %, p=0.03).The difference in the distribution of plasma Lp(a) levels between demented and non-demented APOE ε4 carriers was very similar to the one reported for subjects with or without coronary artery disease (Sandholzer et al, *Arterioscl. Thromb. Vas. Biol*., 1992, 12:1214-1216). In contrast, among non-APOE ε4carriers, median plasma Lp(a) level was similar for subjects with our without dementia [8,6 mg/dl vs 8.5 mg/dl, p = 0.82].This observation suggested that the difference in plasma Lp(a) levels between demented and non-demented APOE ε4 carriers was not due to dementia itself, nor to the age difference between these two groups.

Binding of Lp(a) to receptors which also have APOE as ligand (VLDL-R (Argraves et al, *J. Clin. Invest*., 1997, 100:2170-2181), LRP (März et al, *FEBS*, 1993, 325:271-275) and GP330/megalin (Niemeier et al, *Arterioscler Thromb. Vas. Biol*. 1998 ; La Ferla et al, *J. Clin. Invest*., 1997, 100:310-320) may depend on the size of apolipoprotein(a) [Apo(a) (März et al, above), the distinctive and highly polymorphic glycoprotein covalently attached to apolipoprotein B of LDL to form Lp(a) (Utermann et al, *Science,* 1989, 246:904-910). The size of the Apo(a) isoform is dictated by the number of K4 copies within the *Apo(a)* gene. To address this question, the inventors examined the size of the Apo(a) isoforms in the four groups of subjects. Overall, the proportion of subjects who had no Apo(a) isoform detectable by immunoblot analysis was 10 % (57/581). The frequency distribution of the size of the Apo(a) isoform for subjects who had only one band detectable, or the smaller Apo(a) isoform for subjects with two detectable bands, was similar between the four groups (table hereunder). As expected, an inverse relationship was observed between the size of the Apo(a) isoforms and plasma Lp(a), and this relationship was similar for the four groups under study.

Next, the inventors characterized the (TTTTA)₅₋₁₂ polymorphism located in the 5' flanking region of the *Apo(a)* gene. This polymorphism is in strong linkage disequilibrium with the size of the *Apo(a)* gene and sequences which impact on plasma levels of Lp(a). As was observed in another populations, the most frequent allele had 8 copies of the TTTTA repeat (69,3 %) whereas alleles containing 10 copies (14,9 %), 9 copies (13,9 %), 11 copies(1,6 %) were less frequent. The frequency of alleles containing 5, 6, 7 or 12 copies of the TTTTA repeat was less than 0,5 % in this population. No significant difference in the distribution of the TTTTA alleles was observed between the four groups. Taken together, these analyses indicated that the association between AD and Apo(a) was not restricted to a subset of Apo(a) isoforms or specific alleles at the *Apo(a)* gene.

To determine the risk of having AD in presence of elevated plasma levels of Lp(a), the inventors performed multivariate logistic regression analysis. A threshold of 8.6 ml/dl was chosen, which was the median plasma Lp(a) level for the entire group. No change in OR was observed in non-APOE ε4 carriers over the age examined (figure1). In contrast, in APOE ε4 carriers, the presence of plasma Lp(a) levels > 8.6 mg/ml was associated with a progressive increasing risk of AD which rose from 1.2 [95 % confidence interval, 0.7-2.1] in subjects aged > 65 (n = 68 controls and 143 AD subjects) to 2.1 (1.1-4.1) for subjects aged > 70 years (n = 59 and 94), 5.1 (1.9-13.8) for subjects aged > 75 years (n = 48 and 42) and 15.2 (1.7-135) for subjects aged > 80 (n = 30 and 9). Similar results were obtained when subjects with early onset were excluded from the analysis or when different thresholds for plasma Lp(a) levels were selected.

Lp(a) has been identified in plasma from a restricted number of species including hedgehog, great apes and humans. Apo(a) that circulates in plasma is synthesized by the liver. In addition, Apo(a) transcripts have been identified in monkey testis and brain and, to an lesser extent, in lung and adrenals. However, whether Apo(a) glycoprotein is present in human or monkey brains is not known. To examine whether the association between Lp(a) and AD had a morphological basis, the inventors performed immunohistochemical analysis on 10 human brains with pathologically proven AD-type cortical changes, and five brains with no sign of AD. Apo(a) was immunodetected using mouse monoclonal antibodies IgG-a5 and IgG-a40, which are directed against the N-terminus (K4-type 2) and the C-terminus (K4-type 9) of Apo(a) respectively, and which do not cross-react with plasminogen. The signal was specific, as both antibodies gave the same pattern and no signal was detected in control sections where the primary antibody was omitted, or when an irrelevant antibody was used. In all AD cases, Apo(a) immunoreactivity was detected in senile plaques (figure 2, panel A), in a subset of neurons with neurofibrillary tangles and granulovacuolar degeneration and in reactive microglia and astrocytes (panels B and C). A small proportion of apparently unchanged neurons were also stained (panel C). In addition, a strong Apo(a) immunoreactivity was detected in endothelial cells of leptomeningeal and parenchymal vessels. In brains from patients without AD-type cortical changes, a strong Apo(a) immunoreactivity was observed on endothelial cells whereas only a few neurons showed Apo(a) immunopositivity. These neurons were without histologically detectable changes. These analyses were suggested that Apo(a) may be implicated in neuronal degeneration present in AD.

Various scenarios can be proposed to account for the APOE ε4 specific association between elevated plasma Lp(a) levels and AD. APOE ε4 carriers are at higher risk of developing AD after brain insult like trauma or stroke. On the other hand, elevated plasma levels of Lp(a) have been associated with cerebral infarctions. It is conceivable that such vascular injuries have none or minimal functional consequences in non-APOE ε4 carriers. In contrast, because neuronal plasticity and capacity of regeneration is limited in APOE ε4 carriers, such vascular injuries may progressively lead to the development of AD. As such, this scenario would reinforce the hypothesis of vascular factors in the pathogenesis of AD. It is also conceivable that binding of Lp(a) -or the fraction of Lp(a) particles which carry APOE- may contribute to increased cholesterol delivery to neurons, which may be toxic, as suggested by the beneficial effect for neurons of cholesterol deprivation *in vitro.* Finally, Apo(a) may interfere with other kringle-containing proteins, like urokinase plasminogen activator or thrombin.

In summary, the date are consistent with elevated plasma levels of Lp(a) acting as a modifier trait in the development of APOE ε4 -associated AD.

## Claims

1. A method of predicting an increased risk of a patient having Alzheimer's disease or for a subject of developing AD comprising :
a) assaying a DNA-containing biological sample for the allele of the APOE gene;
b) assaying the plasmatic level of protein Lp(a) or glycoprotein Apo(a),
wherein the presence of an ε4 allele of the APOE gene and an increased plasmatic level of [Lp(a)]/Apo(a) indicates an increased risk for the patient having Alzheimer's disease or developing AD.

2. A method according to claim 1 wherein the level of Lp(a)/Apo(a) is assayed by means of an immunological assay, namely an immunoenzymatic assay.

3. A method according to claim 2 wherein the Lp(a)/Apo(a) level is assayed by means of a sandwich ELISA method carrying out a first antibody against K4-type 1 and 2 repeats (for capturing Lp(a)) and a second antibody conjugated to horseradish peroxidase directed against K4-type 9 repeats for performing the detection of the immune complex formed thereby.

4. A method according to claims 1 to 3 comprising :
i) amplifying the DNA in a DNA-combining biological sample from ma patient or a subject with a first pair of primers which amplify at least or portion of the APOE gene ;
ii) visualizing the amplification products of step i) and thereby assaying for the presence of the APOE ε4 allele.

5. A kit comprising :
- a first pair of primers which are capable of amplifying at least a portion of the APOE gene ;
- a reagent for assaying the presence in a DNA sample of the APOE ε4 allele ;
- at least one antibody recognizing Lp(a)/Apo(a).

6. The kit according to claim 5 wherein the said reagent is a probe specific for APOE ε4.

7. The kit according to claim 5 or 6 further comprising at least one reagent for amplifying DNA.

8. The kit according to any one of claims 5 to 7 further comprising reagents for performing polyacrylamide gel electrophoresis.

9. The kit according to claim 5 wherein said antibody is conjugated to an enzyme, namely horseradish peroxidase.

10. The use of a compound capable of decreasing the plasmatic level of Lp(a) for the manufacture of a medicament for preventing or treating the Alzheimer disease in a subject bearing at least one APO ε4 allele.

11. The use of a compound according to claim 10 wherein said compound modifies the transcription of the Apo(a) gene, and namely consists of danazol or retinoic acid.

12. The use of a compound according to claim 10 wherein said compound modifies the expression of the Apo(a) gene, and namely consists of an oligonucleotide ribozyme.

13. The use of a compound according to claim 10 wherein said compound inhibits the production of the Apo(a)/Lp(a) protein.

14. The use of a compound according to claim 13 wherein said compound modifies the folding of the Apo(a) protein and namely consists of an anti-oxidant inhibiting the formation of disulfide bridges.

15. The use of a compound according to claim 13 wherein said compound modifies the glycosylation of the Apo(a) protein, and namely consist of catanospermine.

16. The use of a compound according to claim 13 wherein said compound modifies the binding of the Apo(a) protein to the cell surface, and namely consists of an antibiotic of the deoxystreptamine family.

17. The use of a compound according to claim 13 wherein said compound modifies the binding of Apo(a) to Apo B100 protein, and namely consists of an analog of lysine, arginine or phenylalanine or of an anti-Apo(a) antibody.

18. The use of a compound capable of inhibiting the binding of Apo(a)/Lp(a) to its receptor for the manufacture of a medicament for preventing or treating the Alzheimer disease in a subject bearing at least one APO ε4 allele.

19. The use according to claim 18 wherein said compound inhibiting the binding of Apo(a)/Lp(a), to its receptor is a compound which blocks the binding site of Apo(a) K4-type 6-7 to the receptor triggered by cholesterol.

20. The use according to claim 18 wherein said compound inhibiting the binding of Apo(a)/Lp(a), to its receptor is a compound which interacts with LPR (low-density lipoprotein receptor related protein), and namely consists of an α-2 macroglobulin or a surface proteoglycan.

## Patentansprüche

1. Verfahren zur Vorhersage eines erhöhten Risikos für einen an der Alzheimer-Krankheit leidenden Patienten oder für eine Person, die Alzheimer-Krankheit zu bekommen, das die
a) Untersuchung einer DNA-haltigen biologischen Probe auf das Allel des Apo-E-Gens und
b) Bestimmung des Plasmaspiegels von Protein Lp(a) oder Glykoprotein Apo(a)
umfasst, wobei das Vorhandensein eines ε4-Allels des Apo-E-Gens und ein erhöhter Plasmaspiegel von [Lp(a)]/Apo(a) ein erhöhtes Risiko für eine Person, die an der Alzheimer-Krankheit leidet oder diese bekommen kann, anzeigt.

2. Verfahren nach Anspruch 1, wobei der Lp(a)/Apo(a)-Spiegel durch einen immunologischen Assay, insbesondere einen immunoenzymatischen Assay, bestimmt wird.

3. Verfahren nach Anspruch 2, wobei der Lp(a)/Apo(a)-Spiegel durch einen Sandwich-ELISA bestimmt wird, worin ein erster Antikörper gegen K4-Typ-1- und -2-repeats (für das Einfangen von Lp(a)) und ein an Meerrettichperoxidase konjugierter zweiter Antikörper, der gegen K4-Typ-9-repeats gerichtet ist, eingesetzt wird, um die Detektion des dadurch gebildeten Immunkomplexes durchzuführen.

4. Verfahren nach den Ansprüchen 1 bis 3, das die
I) Amplifikation der DNA in einer DNA-haltigen biologischen Probe von einem Patienten oder einer Person mit einem ersten Paar Primern, die mindestens einen Teil des Apo-E-Gens amplifizieren, und
II) Sichtbarmachung der Amplifikationsprodukte aus Stufe I) und damit die Untersuchung auf Vorhandensein des Apo-E-ε4-Allels
umfasst.

5. Kit, der
- ein erstes Paar Primer, die in der Lage sind, mindestens einen Teil des Apo-E-Gens zu amplifizieren,
- ein Reagenz für die Untersuchung auf Vorhandensein des Apo-E-ε4-Allels in einer DNA-Probe und
- mindestens einen Lp(a)/Apo(a) erkennenden Antikörper
umfasst.

6. Kit nach Anspruch 5, wobei das Reagenz eine für Apo E ε4 spezifische Sonde ist.

7. Kit nach Anspruch 5 oder 6, der außerdem mindestens ein Reagenz für die Amplifikation der DNA enthält.

8. Kit nach einem der Ansprüche 5 bis 7, der weiterhin Reagenzien für die Durchführung einer Polyacrylamidgelelektrophorese enthält.

9. Kit nach Anspruch 5, wobei der Antikörper an ein Enzym, insbesondere Meerrettichperoxidase, konjugiert ist.

10. Verwendung einer Verbindung, die in der Lage ist, den Plasmaspiegel von Lp(a) zu senken, für die Herstellung eines Arzneimittels zur Verhütung oder Behandlung der Alzheimer-Krankheit bei einer Person, die mindestens ein Apoε4-Allel in sich trägt.

11. Verwendung einer Verbindung nach Anspruch 10, wobei die Verbindung die Transkription des Apo(a)-Gens modifiziert und insbesondere aus Danazol oder Retinsäure besteht.

12. Verwendung einer Verbindung nach Anspruch 10, wobei die Verbindung die Expression des Apo(a)-Gens modifiziert und insbesondere aus einem Oligonucleotidribozym besteht.

13. Verwendung einer Verbindung nach Anspruch 10, wobei die Verbindung die Produktion des Apo(a)/Lp(a)-Proteins inhibiert.

14. Verwendung einer Verbindung nach Anspruch 13, wobei die Verbindung die Faltung des Apo(a)-Proteins modifiziert und insbesondere aus einem die Bildung von Disulfidbrücken inhibierenden Antioxidationsmittel besteht.

15. Verwendung einer Verbindung nach Anspruch 13, wobei die Verbindung die Glykosilierung des Apo(a)-Proteins modifiziert und insbesondere aus Catanospermin besteht.

16. Verwendung einer Verbindung nach Anspruch 13, wobei die Verbindung die Bindung des Apo(a)-Proteins an die Zelloberfläche modifiziert und insbesondere aus einem Antibiotikum der Desoxystreptamin-Familie besteht.

17. Verwendung einer Verbindung nach Anspruch 13, wobei die Verbindung die Bindung von Apo(a) an Apo-B100-Protein modifiziert und insbesondere aus einem Analogon des Lysins, Arginins bzw. Phenylalanins oder einem Anti-Apo(a)-Antikörper besteht.

18. Verwendung einer Verbindung, die in der Lage ist, die Bindung von Apo(a)/Lp(a) an seinen Rezeptor zu inhibieren, zur Herstellung eines Arzneimittels für die Verhütung oder Behandlung der Alzheimer-Krankheit bei einer Person, die mindestens ein Apo-ε4-Allel in sich trägt.

19. Verwendung nach Anspruch 18, wobei die Verbindung, welche die Bindung von Apo(a)/Lp(a) an seinen Rezeptor inhibiert, eine Verbindung ist, welche die Bindungsstelle von Apo(a) K4-Typ 6-7 an den Rezeptor blockiert, der von Cholesterin ausgelöst wird.

20. Verwendung nach Anspruch 18, wobei die Verbindung, welche die Bindung von Apo(a)/Lp(a) an seinen Rezeptor inhibiert, eine Verbindung ist, die mit LPR (mit dem Rezeptor für Lipoprotein niedriger Dichte verwandtes Protein) wechselwirkt und insbesondere aus einem α₂-Makroglobulin oder einem Oberflächenproteoglykan besteht.

## Revendications

1. Procédé pour prédire un risque aggravé pour un patient atteint de la maladie d'Alzheimer ou pour un sujet de développer la maladie d'Alzheimer, comprenant :
a) le dosage sur un prélèvement biologique contenant de l'ADN de l'allèle du gène APOE ;
b) le dosage du taux plasmatique de protéine Lp(a) ou de glycoprotéine Apo(a),
dans lequel la présence d'un allèle ε4 du gène APOE et d'un taux plasmatique accru de [Lp(a)]/Apo(a) indique un risque aggravé pour le patient de contracter la maladie d'Alzheimer ou de développer la maladie d'Alzheimer.

2. Procédé selon la revendication 2 dans lequel le taux de Lp(a)/Apo(a) est dosé au moyen d'un dosage immunologique, précisément d'un dosage immunoenzymatique.

3. Procédé selon la revendication 2 dans lequel le taux de Lp(a)/Apo(a) est dosé au moyen d'une méthode ELISA en sandwich mettant en oeuvre un premier anticorps contre des répétitions K4 de type 1 et 2 (pour capturer Lp(a)) et un second anticorps conjugué à de la peroxydase de raifort contre des répétitions K4 de type 9 pour effectuer la détection du complexe immun ainsi formé.

4. Procédé selon les revendications 1 à 3 comprenant:
i) l'amplification de l'ADN dans un prélèvement biologique contenant de l'ADN d'un patient ou d'un sujet avec une première paire d'amorces qui amplifient au moins une partie du gène APOE;
ii) visualiser les produits d'amplification de l'étape i) et doser ainsi la présence de l'allèle ε4 de APOE.

5. Trousse comprenant :
- une première paire d'amorces qui sont capables d'amplifier au moins une partie du gène APOE;
- un réactif pour doser la présence dans un prélèvement d'ADN de l'allèle ε4 de APOE;
- au moins un anticorps reconnaissant Lp(a)/Apo(a).

6. Trousse selon la revendication 5 dans laquelle ledit réactif est une sonde spécifique de ε4 de APOE.

7. Trousse selon la revendication 5 ou 6 comprenant en outre au moins un réactif pour amplifier l'ADN.

8. Trousse selon l'une quelconque des revendications 5 à 7 comprenant en outre des réactifs pour réaliser une électrophorèse sur gel de polyacrylamide.

9. Trousse selon la revendication 5 dans laquelle ledit anticorps est conjugué à une enzyme, précisément à la peroxydase de raifort.

10. Utilisation d'un composé capable d'abaisser le taux plasmatique de Lp(a) pour la fabrication d'un médicament destiné à la prévention ou au traitement de la maladie d'Alzheimer chez un sujet porteur d'au moins un allèle ε4 de APOE.

11. Utilisation d'un composé selon la revendication 10 dans laquelle ledit composé modifie la transcription du gène Apo(a) et se compose précisément du danazol ou de l'acide rétinoïque.

12. Utilisation d'un composé selon la revendication 10 dans laquelle ledit composé modifie l'expression du gène Apo(a), et se compose précisément d'un oligonucléotide ribozyme.

13. Utilisation d'un composé selon la revendication 10 dans laquelle ledit composé inhibe la production de la protéine Apo(a)/Lp(a).

14. Utilisation d'un composé selon la revendication 13 dans laquelle ledit composé modifie le repliement de la protéine Apo(a) et se compose précisément d'un antioxydant inhibant la formation de ponts disulfure.

15. Utilisation d'un composé selon la revendication 13 dans laquelle ledit composé modifie la glycosylation de la protéine Apo(a) et se compose précisément de la catanospermine.

16. Utilisation d'un composé selon la revendication 13 dans laquelle ledit composé modifie la liaison de la protéine Apo(a) à la surface cellulaire et se compose précisément d'un antibiotique de la famille des désoxystreptamines.

17. Utilisation d'un composé selon la revendication 13 dans laquelle ledit composé modifie la liaison de la protéine Apo(a) à la protéine Apo B100 et se compose précisément d'un analogue de lysine, d'arginine ou de phénylalanine ou d'un anticorps anti-Apo(a).

18. Utilisation d'un composé capable d'inhiber la liaison de Apo(a)/Lp(a) à son récepteur pour la fabrication d'un médicament destiné à la prévention ou au traitement de la maladie d'Alzheimer chez un sujet porteur d'au moins un allèle ε4 de APOE.

19. Utilisation selon la revendication 18 dans laquelle ledit composé inhibant la liaison de Apo(a)/Lp(a) à son récepteur est un composé qui bloque le site de liaison K4 de type 6-7 de Apo(a) au récepteur déclenché par le cholestérol.

20. Utilisation selon la revendication 18 dans laquelle ledit composé inhibant la liaison de Apo(a)/Lp(a) à son récepteur est un composé qui interagit avec la LPR (protéine de la famille des récepteurs de lipoprotéine de basse densité) et se compose précisément d'une α-2-macroglobuline ou d'un protéoglycane de surface.
